(19) Europäisches Patentamt
European Patent Office
Office européen des brevets

(11) Numéro de publication: **0 054 442**
**B1**

# (12) FASCICULE DE BREVET EUROPEEN

(45) Date de publication du fascicule du brevet: **29.08.84**

(21) Numéro de dépôt: **81401668.9**

(22) Date de dépôt: **22.10.81**

(51) Int. Cl.³: **C 07 D 495/04,** A 61 K 31/435 // C07F7/10, C07F5/02, (C07D495/04, 333/00, 221/00)

(54) **Dérivés de la thiéno-pyridinone, leur procédé de préparation et leur application thérapeutique.**

(30) Priorité: **28.11.80 FR 8025274**

(43) Date de publication de la demande: **23.06.82 Bulletin 82/25**

(45) Mention de la délivrance du brevet: **29.08.84 Bulletin 84/35**

(84) Etats contractants désignés: **AT BE CH DE GB IT LI LU NL SE**

(56) Documents cités: **FR - A - 2 215 948**

(73) Titulaire: **SANOFI, Société dite:, 40, Avenue George V, F-75008 Paris (FR)**

(72) Inventeur: **Boigegrain, Robert, 28 Ancienne Route Impériale, F-31120 Portet-Sur-Garonne (FR)**
Inventeur: **Maffrand, Jean-Pierre, 5 Rue du Corps-Franc Pommiès, F-31120 Portet-Sur-Garonne (FR)**
Inventeur: **Suzuki, Norio, 942-116 Naganumahara-cho, Chiba-Shi Chiba (JP)**
Inventeur: **Matsubayashi, Kiuichi, 21-22 Yagigaya-cho, Funabashi-Shi Chiba (JP)**
Inventeur: **Ashida, Shinichiro, 1-12 Katemama, 2-chome, Ichikawa-Shi Chiba (JP)**

(74) Mandataire: **Polus, Camille et al, c/o Cabinet Lavoix 2, Place d'Estienne d'Orves, F-75441 Paris Cedex 09 (FR)**

**EP 0 054 442 B1**

Il est rappelé que Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à de nouveaux dérivés de tétrahydro-5, 6, 7, 7a 4H-thiéno (3,2-c) pyridinones-2, à leur procédé de préparation et à leur application en médecine humaine et vétérinaire.

Les composés selon l'invention répondent à la formule générale suivante:

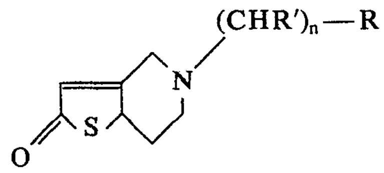

(I)

dans laquelle

R représente l'hydrogène ou un radical phényle éventuellement substitué par au moins un atome d'halogène ou un groupe alcoyle en $C_1-C_4$, alcoxy en $C_1-C_4$, nitro, carboxy, alcoxycarbonyle ou cyano;

R' représente l'hydrogène ou un groupe alcoyle inférieur; et

n est 0 ou un nombre entier de 1 à 4;

ainsi que leurs sels d'addition avec les acides minéraux ou organiques pharmaceutiquement acceptables.

Ces composés, comportant un ou plusieurs atomes de carbone asymétriques peuvent exister sous forme de plusieurs stéréoisomères (énantiomères ou diastéréoisoères). L'invention concerne aussi bien chaque stéréoisomère que leur mélange.

Ces composés qui présentent des propriétés anti-agrégantes plaquettaires et anti-thrombotiques sont inclus dans une formule générale revendiquée dans les brevets français N° 2.215.948 et 2.345.150, sous leur forme tautomère suivante:

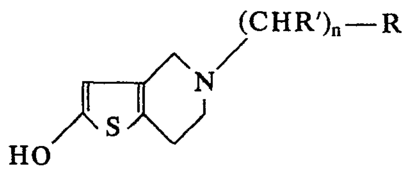

Cependant aucun de ces produits n'est spécifiquement décrit.

En outre, l'étude toxicologique et pharmacologique des composés de l'invention a mis en évidence des propriétés particulières tant sur le plan de l'efficacité et de la tolérance, que de la nature même de ces propriétés.

L'invention a également pour objet un procédé de préparation des composés de formule I définie ci-dessus, caractérisé en ce que:

a) on oxyde un dérivé boronique de formule:

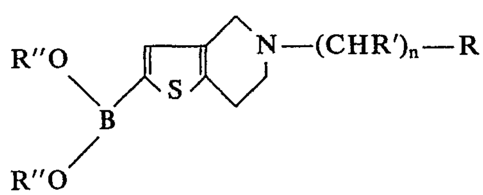

(V)

dans laquelle R, R' et n ont les significations indiquées pour la formule I mentionnée ci-dessus, et R'' est un atome d'hydrogène ou un groupe alkyle inférieur; et

b) on hydrolyse le dérivé borique obtenu de formulee suivante:

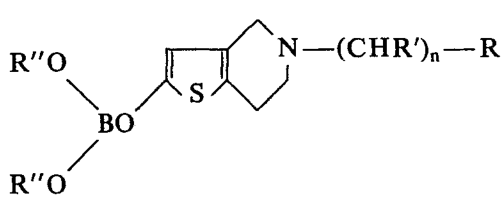

(VI)

dans laquelle R, R', R'' et n sont tels que définis ci-dessus, pour obtenir le dérivé de formule I.

L'oxydation du dérivé boronique (V) qui peut être un boronate (Va) ou un acide boronique (V) qui peut être un boronate (Va) ou un acide boronique (Vb) est réalisée dans un solvant inerte et en empêchant une élévation de température du milieu réactionnel.

La préparation du dérivé boronique de formule (V) peut être réalisée par deux variantes décrites ci-après selon qu'il s'agit du boronate (Va) ou de l'acide boronique (Vb), ces deux variantes possèdant un tronc préalable commun.

Selon le tronc commun, on fait réagir un composé de formule (II) dans laquelle X est le groupe $-(CHR')_nR$ tel que défini précédemment ou le groupe triméthylsilyle, avec un alkyllithium tel que le butyllithium ou un amidure de lithium tel que le diisopropylamidure de lithium, pour obtenir ainsi un dérivé lithié (III) que l'on condense dans le même récipient réactionnel avec un borate d'alkyle de formule $B(OR''')_3$ dans laquelle R''' est un radical alkyle inférieur, tel que le borate de n-butyle, pour préparer un boronate de formule (IV), selon le schéma réactionnel ci-après:

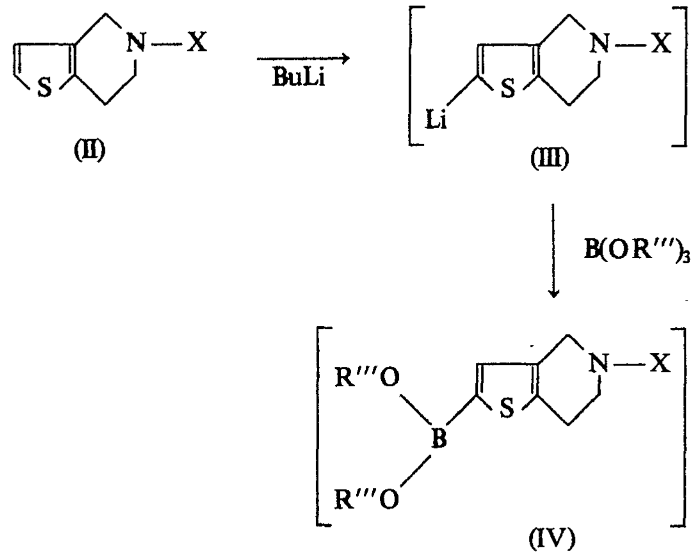


(II) (III) (IV)

La réaction de lithiation a lieu dans un solvant inerte tel que l'éther, le tétrahydrofuranne, l'hexane, éventuellement en présence d'un agent complexant tel que l'hexaméthylphosphotriamide, à une température comprise entre −50° C et +30° C.

L'addition de borate s'effectue entre 0° et −80° C, puis on laisse remonter la température éventuellement jusquà la température ambiante.

Selon une première variante, dans le cas où X représente le groupe $-(CHR')_n-R$, le boronate de formule (Va) dans lequel R'' est un groupe alkyle inférieur, est traité à 30 p. 100 d'eau oxygénée. On obtient alors un borate de formule (VIa) dont l'hydrolyse immédiate dans le milieu conduit au composé de formule (I), selon le schéma réactionnel suivant:

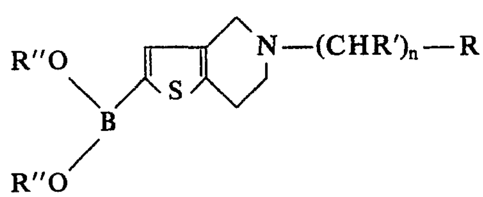

(Va)

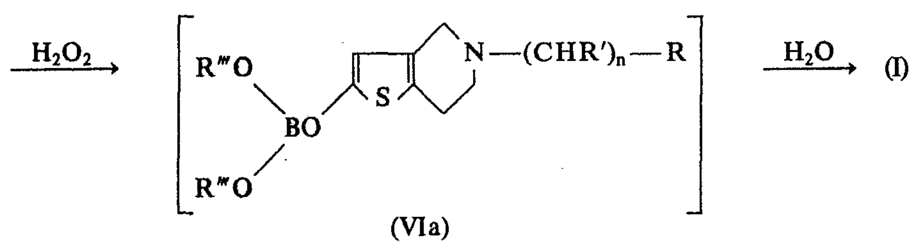


(VIa)

Selon une seconde variante obtenue à l'issue du tronc commun on traite le boronate de formule (IV), dans lequel X représente le groupe triméthylsilyle, par de l'acide chlorhydrique 3N selon le schéma suivant:

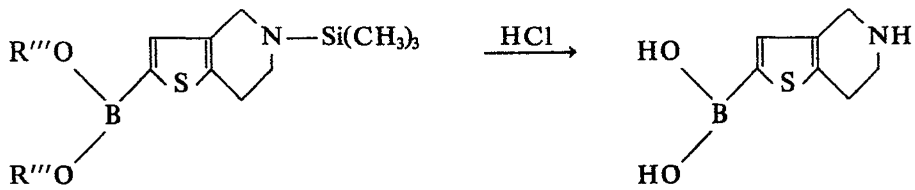


(IV) (VII)

L'acide boronique de formule (VII) obtenu est ensuite alkylé avec un composé de formule $R(CHR')_n—Y$ dans laquelle R, R' et n ont les significations précédentes et Y est un halogène, de préférence le chlore, le brome ou l'iode, ou un radical arylsulfonyloxy tel que paratoluènesulfonyloxy ou benzènesulfonyloxy ou un radical alkylsulfonyloxy, tel que méthanesulfonyloxy, pour ainsi obtenir un dérivé hygroscopique qui est un acide boronique de formule (Vb) qu'il n'est pas nécessaire de purifier avant de le transformer, selon le même processus réactionnel que pour le boronate de formule (Va) de la variante précédente, en dérivé borique de formule (VIb), puis en composé de formule (I) par traitement, tout d'abord avec une solution aqueuse d'eau oxygénée, puis hydrolyse aqueuse, selon le schéma réactionnel ci-dessous:

(VII)

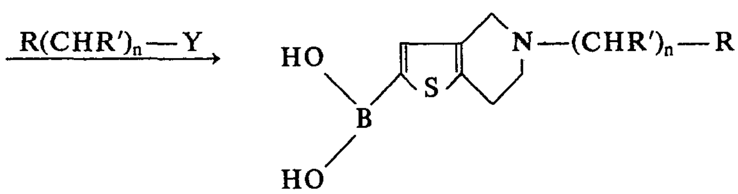


(Vb)

(Vb)

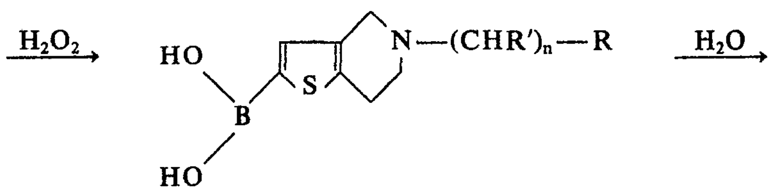


(I)

(VIb)

La condensation du dérivé (VII) avec l'agent alkylant $R(CHR')_n—Y$ s'effectue dans un solvant inerte tel qu'un alcanol inférieur, le tétrahydrofuranne, le dioxanne ou le diméthylformamide, en présence d'une base telle que le carbonate de sodium ou de potassium, pour neutraliser l'acide HY libéré. Il est préférable d'opérer à des températures comprises entre 50° C et la température d'ébullition du milieu.

L'oxydation de l'acide boronique (Vb) par l'eau oxygénée s'effectue à une température comprise entre 0° C et 10° C dans un solvant inerte tel que le tétrahydrofuranne ou le dioxanne.

Le composé de formule (II) dans lequel X représente le groupe triméthylsilyle et le composé de formule (VII) qui sont utilisés à titre d'intermédiaires dans le procédé de l'invention sont nouveaux.

Le composé de formule II dans lequel X représente le groupe triméthylsilyle est préparé par condensation sur le tétrahydro-4,5,6,7 thiéno (3,2-c) pyridine de chlorotriméthylsilane en présence d'une base organique comme accepteur d'acide et dans un solvant inerte. On réalise de préférence cette réaction de condensation à chaud.

Les exemples non limitatifs suivants illustrent l'invention:

## Exemple 1

o-chlorobenzyl-5 tétrahydro-5, 6, 7, 7a 4H-thiéno (3,2-c) pyridinone-2,
formule I, R = 2 — Cl — $C_6H_4$; R = H; n = 1; dérivé n° 1

A une solution refroidie à — 20° C de 32,6 g (0,123 Mole) d'o-chlorobenzyl-5 tétrahydro-4,5,6,7 thiéno (3,2-c) pyridine dans 320 $cm^3$ de tétrahydrofuranne (T.H.F.), on additionne, goutte à goutte, 79 $cm^3$ d'une solution de butyllithium à 12% dans l'hexane (0,147 mole). A la fin de l'addition le lithien (III) précipite et on laisse revenir la température à 0° C. On ajoute alors 15 $cm^3$ d'hexaméthylphosphotriamide (HMPT) préalablement séché sur tamis moléculaire 4 Å. Le précipité devient rouge foncé.

La température est abaissée à —40° C et on ajoute, goutte à goutte, en une demi-heure, une solution de borate de tributyle (39,8 $cm^3$; 0,147 mole) dans 40 $cm^3$ de tétrahydrofuranne anhydre. Le précipité disparait et le milieu réactionnel devient jaune clair. La température est maintenue à —40° C pendant une demi-heure puis ramenée à 10° C (2 heures). On ajoute, goutte à goutte, 33 $cm^3$ (0,291 mole) d'eau oxygénée à 30% en maintenant la température du milieu inférieure à 30° C. Un précipité intense se forme en cours d'addition. L'agitation est maintenue pendant 1 heure à température ambiante. On verse le milieu réactionnel sur de l'eau, extrait par 3 × 200 $cm^3$ d'éther éthylique, sèche les phases organiques sur sulfate de sodium et concentre sous vide à température inférieure à 40° C. Le liquide résiduel est chromatographié sur colonne de silice (cyclohexane-acétate d'éthyle 6/4) pour éliminer le HMPT restant. Après évaporation, on traite par un équivalent molaire d'acide oxalique dans l'acétone, et filtre les cristaux jaune clair formés.

Par recristallisation dans l'éthanol, on obtient des cristaux beiges d'oxalate;
rendement: 52%, F: 170° C, IR (KBr): $\nu$ CO: 1660 $cm^{-1}$ (large).
Base:
F: 73° — 74,5° C (éthanol).
RMN ($CDCl_3$):
7,1 — 7,6 (m,4H); 6,2 (s,1H); 4,2 — 4,7 (m,1H); 3,9 (s,2H); 1,5 — 4,2 (m,6H).
Chlorhydrate, hemihydrate:
F: décomposition vers 180° C (précipitation dans l'acétone).

## Exemple 2

Benzyl-5 tétrahydro-5,6,7,7a 4H-thiéno (3,2-c) pyridinone-2,
Formule I, R = $C_6H_5$; R' = H; n = 1; dérivé n° 2

On le prépare selon le mode de l'exemple 1 à partir de la benzyl-5 tetrahydro-4,5,6,7 thiéno (3,2-c)pyridine.
Maléate:
cristaux beiges, F = 132 — 134° C (isopropanol); rendement: 33%. IR (KBr): $\nu$ CO: 1680 $cm^{-1}$.
Base:
RMN ($CDCl_3$: 7,25 (m,5H); 5,90 (s,1H); 3,60 (s,2H).

## Exemple 3

p-Chlorobenzyl-5 tétrahydro-5,6,7,7a 4H-thiéno (3,2-c) pyridinone-2,
Formule I, R = 4 — Cl — $C_6H_4$; R' = H; n = 1; dérivé n° 3

On le prépare selon le mode opératoire de l'exemple 1 à partir de la p-chlorobenzyl-5 tétrahydro-4,5,6,7 thiéno (3,2-c) pyridine.

Maléate:
cristaux beiges, F = 158 — 160° C (éthanol); rendement: 42%. IR (KBr): $\nu$ CO = 1680 $cm^{-1}$.
Base:
RMN ($CDCl_3$): 7,30 (m,4H); 6,0 (s,1H), 3,50 (s,2H).

Exemple 4

o-méthylbenzyl-5 tétrahydro-5,6,7,7a 4H-thiéno (3,2-c) pyridinone-2,
Formule I, $R=2-CH_3-C_6H_4-$; $R'=H$; $n=1$; dérivé n° 4

On le prépare selon le mode opératoire de l'exemple 1 à partir de l'o-méthylbenzyl-5 tétrahydro-4,5,6,7 thiéno (3,2-c) pyridine.

Oxalate:
cristaux beiges; F = 195—197° C (méthanol); rendement: 33%. IR (KBr): $\nu$ CO = 1690 $cm^{-1}$.
Base:
RMN ($CDCl_3$): 7,10 (s,4H); 5,90 (s,1H); 3,55 (s,2H); 2,30 (s,3H).

Exemple 5

[(Chloro-2 phényl)-1 éthyl]-5 tétrahydro-5,6,7,7a 4H-thiéno (3,2-c) pyridinone-2,
Formule (I), $R=2-Cl-C_6H_4$; $R'=CH_3$; $n=1$; dérivé n° 5

On le prépare selon le mode opératoire de l'exemple 1 à partir de la [(chloro-2 phényl)-1 éthyl]-5 tétrahydro-4,5,6,7 thiéno (3,2-c) pyridine

Chlorhydrate:
cristaux jaunes, F = 140—142° C; rendement: 24% IR (KBr): $\nu$ CO = 1690 $cm^{-1}$.
Base:
RMN ($CDCl_3$): 7,30 (m,4H); 6,05 et 5,95 (2s, 1H) (2 diastéréoisomères).

Exemple 6

[(Chloro-2 phényl)-1 propyl]-5 tétrahydro-5,6,7,7a 4H-thiéno (3,2-c) pyridinone-2,
Formule (I), $R=2-Cl-C_6H_4$; $R'=C_2H_5$; $n=1$; dérivé n° 6

On le prépare selon le mode opératoire de l'exemple 1 à partir de la [(chloro-2 phényl)-1 propyl]-5 tétrahydro-4,5,6,7 thiéno (3,2-c) pyridine.

Chlorhydrate:
cristaux beiges, F = 124—126° C; rendement: 27%. IR (KBr): $\nu$ CO = 1690 $cm^{-1}$.
Base:
RMN ($CDCl_3$): 7,30 (m,4H); 6,05 et 5,90 (2s,1H) (2 diastéréoisomères).

Exemple 7

Triméthylsilyl-5 tétrahydro-4,5,6,7 thiéno (3,2-c) pyridine, de formule (II):
$X=(CH_3)_3Si$

A une solution de 80 g (0,571 mole) de tétrahydro-4,5,6,7 thiéno (3,2-c) pyridine et de 63,4 g (0,28 mole) de triéthylamine dans 1100 $cm^3$ de toluène, on additionne, sous atmosphère d'azote, 65 g (0,628 mole) de chlorotriméthylsilane dans 50 $cm^3$ de toluène. Le milieu réactionnel est porté à 80° C pendant 3 heures. Après refroidissement le précipité blanc de chlorhydrate de triéthylamine est filtré et le filtrat est concentré sous vide.

Le résidu est distillé à 60° —70° C sous 0,1 mmHg.

Liquide incolore, rendement: 55%.

Exemple 8

Acide tétrahydro-4,5,6,7 thiéno (3,2-c) pyridine boronique-2, de formule (VII)

A une solution refroidie à —20° C de 15 g (0,07 mole) de triméthylsilyl-5 tétrahydro-4,5,6,7 thiéno (3,2-c) pyridine, préparée à l'exemple 7, dans 150 $cm^3$ de THF, on additionne, goutte à goutte, sous atmosphère d'azote, 45,4 $cm^3$ d'une solution à 12% de butyllithium dans l'hexane (0,084 mole). On laisse revenir la température à 0° C et on ajoute 3 $cm^3$ de HMPT. Après refroidissement à —50° C on additionne, goutte à goutte, une solution de 19,3 g (0,084 mole) de borate de tributyle dans 30 $cm^3$ de

THF. l'agitation est maintenue pendant 2 heures, en laissant revenir à température ambiante. On ajoute ensuite 28 $cm^3$ d'acide chlorhydrique 3N (0,084 mole), et on filtre le précipité formé. Les cristaux sont lavés à l'acétone et à l'éther diisopropylique, puis séchés sous vide.

Cristaux blanc cassé:

F > 260° C; rendement: quantitatif.

RMN ($D_2O$):

6,75 (s,1H); 4,10 (m,2H); 2,80—3,50 (m,4H).

Exemple 9

Acide o-cyanobenzyl-5 tétrahydro-4,5,6,7 thiéno (3,2-c) pyridine boronique-2
Formule (Vb), R = 2—CN—$C_6H_4$; R' = H; n = 1

On chauffe à 80° C pendant 3 heures un mélange de 3,68 g (0,02 mole) d'acide tétrahydro-4,5,6,7 thiéno (3,2-c) pyridine boronique-2 préparé à l'exemple 8, 9,09 g (0,06 mole) de chlorure d'o-cyanobenzyle et 5,52 g (0,04 mole) de carbonate de potassium dans 40 $cm^3$ de diméthylformamide. Après évaporation du solvant, le milieu réactionnel est additionné d'eau puis extrait par 3 fois 100 $cm^3$ de chlorure de méthylène. La solution organique est séchée sur sulfate de sodium puis évaporée sous vide. Les cristaux obtenus sont lavés à l'éther diisopropylique.

Cristaux blanc cassé:

F = 140° — 142° C; rendement: 45%.

IR (KBr):

$\nu$ CN = 2220 $cm^{-1}$.

RMN (DMSO $D_6$):

7,60 (m,4H); 7,25 (s,1H); 3,80 (s,2H); 3,50 (s,2H); 2,80 (s,4H).

Exemple 10

o-cyanobenzyl-5 tétrahydro-5,6,7,7a 4H-thiéno (3,2-c) pyridinone-2,
Formule (I), R = 2—CN—$C_6H_4$; R' = H; n = 1; dérivé n° 7

A une solution refroidie à 5° C de 1,8 g (0,006 mole) d'acide o-cyanobenzyl-5 tétrahydro-4,5,6,7 thiéno (3,2-c) pyridine boronique-2, préparé à l'exemple 9, dans 30 $cm^3$ de tétrahydrofuranne, on ajoute, goutte à goutte, 0,23 g (0,006 mole) d'une solution d'eau oxygénée à 30%. La température est ramenée à l'ambiante, et l'agitation maintenue pendant 2 heures. Le milieu est additionné d'eau puis extrait au chlorure de méthylène. La solution organique est séchée sur sulfate de sodium puis concentrée sous vide. Le résidu est chromatographié sur colonne de silice (cyclohexaneacétate d'éthyle 1/1). Après évaporation on traite par un équivalent molaire d'acide oxalique dans l'acétone et filtre les cristaux formés.

Oxalate:

cristaux beiges; F = 176° — 178° C (acétonitrile); rendement: 28%.

IR (KBr):

$\nu$ CO: 1700 $cm^{-1}$; $\nu$ CN: 2210 $cm^{-1}$.

Base:

RMN ($CDCl_3$): 7,50 (m,4H); 6,00 (s,1H); 3,80 (s,2H).

Exemple 11

o-nitrobenzyl-5 tétrahydro-5,6,7,7a 4H-thiéno (3,2-c) pyridinone-2,
Formule (I), R: 2—$NO_2$—$C_6H_4$; R' = H; n = 1; dérivé n° 8

a) préparation de l'acide o-nitrobenzyl-5 tétrahydro-4,5,6,7 thiéno (3,2-c)
pyridine boronique-2,
Formule (Vb), R = 2—$NO_2$—$C_6H_4$; R' = H; n = 1

On le prépare selon le mode opératoire de l'exemple 9 à partir de l'acide tétrahydro-4,5,6,7 thiéno (3,2-c) pyridine boronique-2 et du chlorure d'o-nitrobenzyle.

Cristaux bruns:

F = 132° — 134° C; rendement: 40%.

RMN (DMSO $D_6$):
8,0 (m,4H); 7,50 (s,1H); 4,00 (s,2H); 3,60 (s,2H); 2,70 (m,4H).

b) préparation du dérivé n° 8

On le prépare selon le mode opératoire de l'exemple 10 à partir de l'acide o-nitrobenzyl-5 tétrahydro-4,5,6,7 thiéno (3,2-c) pyridine boronique-2 ci-dessus.

Oxalate:
cristaux beiges; F = 186° — 188° C (isopropanol-éthanol), rendement: 17%.
IR:
$\nu$ CO = 1685 $cm^{-1}$.
Base:
RMN ($CDCl_3$) 7,50 (m,4H); 5,95 (s,1H); 3,90 (s,2H).

Exemple 12

o-bromobenzyl-5 tétrahydro-5,6,7,7a 4H-thiéno (3,2-c) pyridinone-2,
Formule (I), R = 2—Br—$C_6H_4$; R' = H; n = 1; dérivé n° 9

a) préparation de l'acide o-bromobenzyl-5 tétrahydro-4,5,6,7 thiéno (3,2-c) pyridine boronique-2,

Formule (Vb), R = 2—Br—$C_6H_4$; R' = H, n = 1

On le prépare selon le mode opératoire de l'exemple 9 à partir de l'acide tétrahydro-4,5,6,7 thiéno (3,2-c) pyridine boronique-2 et du bromure d'o-bromobenzyle.

Cristaux jaunes:
F = 129° — 131° C; rendement: 53%.
RMN (DMSO $D_6$):
7,50 (m,5H); 3,70 (m,2H); 3,10 (s,2H); 2,80 (m,4H).

b) préparation du dérivé n° 9

On le prépare selon le mode opératoire de l'exemple 10 à partir de l'acide o-bromobenzyl-5 tétrahydro-4,5,6,7 thiéno (3,2-c) pyridine boronique-2 ci-dessus.

Oxalate:
cristaux beiges; F = 151° — 153° C (isopropanol); rendement: 5%.
IR (KBr):
$\nu$ CO = 1690 $cm^{-1}$.
Base:
RMN ($CDCl_3$) 7,30 (m,4H); 5,95 (s,1H); 3,75 (s,2H).

Les résultats des essais toxicologiques et pharmacologiques qui sont rapportés ci-après, ont permis de mettre en évidence les intéressantes propriétés des dérivés de l'invention sur les plans toxicologique et pharmacologique. Ces études ont été effectuées comparativement aux composés les plus représentatifs des deux brevets cités plus haut, c'est-à-dire la (chloro-2 benzyl)-5 tétrahydro-4,5,6,7 thiéno (3,2-c) pyridine appelée ci-après composé de référence A (composé n° 1 du brevet francais n° 2.215.948) et la (cyano-2 benzyl)-5 tétrahydro-4,5,6,7 thiéno (3,2-c) pyridine appelée ci-après composé de formule B (composé n° 8 du brevet français n° 2.345.150).

L'invention a donc encore pour objet un médicament ayant en particulier des propriétés anti-agrégante plaquettaire et anti-thrombotique, caractérisé en ce qu'il contient, à titre de principe actif, un dérivé de formule (I) ou un sel d'addition avec un acide minéral ou organique pharmaceutiquement acceptable.

Etude toxicologique

Cette étude a porté sur les toxicités aigüe, chronique, subchronique et retardée. Les essais, effectués sur différentes espèces animales: souris, rats et lapins, ont mis en évidence la faible toxicité des composés de l'invention ainsi que leur bonne tolérance.

A titre indicatif, la DL 50/24 h/kg de poids corporel, calculée selon la méthode de Miller et Tainter, par la voie intraveineuse, chez la souris est donnée au tableau I ci-après qui rassemble les résultats des dérivés de l'invention et ceux des composés de référence A et B. L'étude de ces résultats montre que les dérivés de formule (I) ont une toxicité au moins deux fois plus faible que les composés de référence A et B.

Tableau I

| Composes | DL 50 |
|---|---|
| n° 1 | 113 mg |
| n° 2 | 116 mg |
| n° 3 | 125 mg |
| n° 4 | 110 mg |
| n° 5 | 286 mg |
| n° 6 | 121 mg |
| n° 7 | 254 mg |
| n° 8 | 278 mg |
| n° 9 | 118 mg |
| Composé de référence A | 55 mg |
| Composé de référence B | 45 mg |

Les essais ont montré, en outre, que les dérivés de l'invention n'ont provoqué chez les animaux des différentes espèces animales tout au long des différents essais pratiqués, aucune réaction locale ou générale, aucune perturbation dans les contrôles biologiques régulièrement effectués, aucune lésion microscopique ou macroscopique.

L'étude de la descendance n'a montré aucun effet tératogène.

Etude pharmacologique

Elle a porté sur l'action inhibitrice de l'agrégation plaquettaire et sur l'activité anti-thrombotique, par comparaison avec les composés de référence A et B.

1. Action inhibitrice de l'agrégation plaquettaire

Chez des rats de souche Wistar, préalablement traités par le composé à tester, on effectue un prélèvement sanguin dans le veine jugulaire. A partir de ce sang, ci-traté et après centrifugation, on reconstitue un plasma contenant $600\,000 \pm 20\,000$ plaquettes par $mm^3$, qui servira dans toutes les mesures d'agrégation.

a) mesure de l'agrégation plaquettaire à l'A.D.P.

On place 0,4 ml de plasma dans un tube siliconé pourvu d'une barre aimantée elle-même siliconée. Le tube est introduit dans un agrégomètre couplé à un appareil permettant d'enregistrer les variations de densité optique. Lorsque la transmission de la lumière a atteint une valeur stable, on introduit dans le tube 0,5 ml d'une solution contenant 10 µM d'A.D.P. (Adénosine-Di Phosphate).

L'agrégation des plaquettes provoque alors une augmentation de la transmission lumineuse suivie d'une diminution consécutive à la phase de désagrégation.

La variation maximale de densité optique ainsi déterminée par rapport à celle d'un plasma déplaquetté caractérise l'intensité de l'agrégation.

Les mesures sont effectuées dans les 2 heures qui suivent le prélèvement sanguin effectué 3 heures après le traitement par le dérivé à tester.

b) mesure de l'agrégation plaquettaire au collagène

La solution d'A.D.P. est remplacée par une solution de collagène (extrait de tendons bovins).

c) résultats

Différents lots de 20 rats ont été utilisés, chaque lot recevant une dose unique du dérivé à tester par la voie orale, le même dérivé étant administré à doses différentes allant de 5 mg/kg à 100 mg/kg.

L'activité significative des dérivés de formule (I) apparaît à la dose de 12,5 mg/kg, alors que pour les composés de référence A et B il faut atteindre des doses de 100 mg/kg pour obtenir une activité similaire.

Les résultats sont consignés dans les tablaux II et III suivants qui indiquent le pourcentage d'inhibition de l'agrégation plaquettaire obtenu par rapport au témoin, 3 heures après le traitement par le composé à tester.

Tableau II

Test à L'A.D.P.

| | Pourcentage d'inhibition 5 mg/kg | 12,5 mg/kg | 25 mg/kg | 50 mg/kg | 100 mg/kg |
|---|---|---|---|---|---|
| Dérivé 1 | 51,0 | 83,6 | 83,7 | 84,0 | 84,1 |
| Dérivé 2 | 47,8 | 82,6 | 82,9 | 83,7 | 83,7 |
| Dérivé 3 | 48,1 | 82,4 | 83,0 | 83,5 | 83,6 |
| Dérivé 4 | 48,0 | 81,9 | 82,1 | 82,8 | 82,9 |
| Dérivé 5 | 48,4 | 82,0 | 82,4 | 83,2 | 83,5 |
| Dérivé 6 | 48,7 | 83,0 | 83,4 | 83,9 | 83,9 |
| Dérivé 7 | 47,6 | 82,7 | 82,9 | 83,1 | 83,2 |
| Dérivé 8 | 48,0 | 82,8 | 83,1 | 83,3 | 83,4 |
| Dérivé 9 | 48,4 | 83,1 | 83,4 | 83,7 | 84,0 |
| Composé de référence A | 0 | 0 | 0 | 34,6 | 63,2 |
| Composé de référence B | 0 | 0 | 0 | 35,5 | 63,8 |

Tableau III

Test au collagène

| | Pourcentage d'inhibition 5 mg/kg | 12,5 mg/kg | 25 mg/kg | 50 mg/kg | 100 mg/kg |
|---|---|---|---|---|---|
| Dérivé 1 | 16,8 | 46,8 | 86,7 | 89,6 | 89,8 |
| Dérivé 2 | 16,5 | 44,2 | 85,8 | 87,0 | 87,8 |
| Dérivé 3 | 17,4 | 44,9 | 86,5 | 87,2 | 88,1 |
| Dérivé 4 | 17,1 | 45,2 | 85,9 | 88,1 | 88,2 |
| Dérivé 5 | 17,0 | 44,8 | 86,1 | 87,8 | 88,0 |
| Dérivé 6 | 16,8 | 44,4 | 85,7 | 89,0 | 89,2 |
| Dérivé 7 | 16,5 | 44,5 | 86,2 | 87,9 | 89,4 |
| Dérivé 8 | 17,0 | 44,6 | 86,2 | 88,2 | 88,7 |
| Dérivé 9 | 17,2 | 44,9 | 86,4 | 87,9 | 88,6 |
| Composé de référence A | 0 | 0 | 40,9 | 46,8 | 80,1 |
| Composé de référence B | 0 | 0 | 38,5 | 51,2 | 78,8 |

d) Étude cinétique de l'agrégation plaquettaire

Une autre expérimentation a été effectuée, portant sur l'étude cinétique des composés de l'invention: le dérivé n° 1 et le composé de référence A sont mis en solution dans du propylèneglycol et administrés à des rats à la dose de 100 mg/kg de poids corporel par la voie intrapéritonéale, les souris témoins ne recevant, par la même voie, que 1 ml/kg de propylèneglycol.

Le sang est prélevé à la 10e minute et à la 60e minute après cette administration, puis centrifugé pour constituer un plasma enrichi en plaquettes.

L'agrégation des plaquettes dans le plasma, induite par l'A.D.P. est mesurée à l'agrégomètre de BRYSTON, selon la méthode néphélométrique de BORN.

Les pourcentages d'inhibition en fonction du temps, obtenus lors de cette expérimentation sont rassemblés dans le tableau IV.

Tableau IV

| Temps en minutes | Dérivé de l'invention n° 1 | Composé de référence A | Témoin (propylèneglycol) |
|---|---|---|---|
| 10 | 42 | 22 | 0 |
| 60 | 97 | 39 | 0 |

Ce tableau met en évidence que, par rapport au composé de référence A, le dérivé de l'invention possède une activité inhibitrice de l'agrégation plaquettaire beaucoup plus intense et que cette activité s'installe aussi plus rapidement; cette expérimentation confirme les résultats décris ci-dessus.

2. Activité anti-thrombotique

Cette activité a été étudiée selon la méthode de la thrombose expérimentale par circulation extracorporelle, décrite par TERUHIKO UMETSU et KAZUKO SONOI (THROMBOS. HAEMOST. 39,1, [1978]).

Sur le rat anesthésié par une injection intrapéritonéale de pentobarbital, la jugulaire gauche et la carotide externe droite sont mises à nu. Le shunt est constitué par une cathéter central et deux cathéters latéraux; un fil de soie naturelle blanche est introduit dans la partie centrale et la circulation rétablie pendant 20 minutes.

Après arrêt de la circulation par clampage, le fil est retiré doucement et pesé immédiatement.

Le poids moyen d'un fil de soie humide a été déterminé au préalable: il est de 5,10 mg.

Les traitements sont pratiqués 48 heures, 24 heures et 2 heures avant le début de la circulation sanguine dans le shunt.

Les produits sont administrés per os, à différents lots de vingt animaux, en suspension dans 10 ml/kg de gomme arabique à 5%, aux doses de 12,5 mg/kg, 25 mg/kg, 50 mg/kg, 100 mg/kg et 200 mg/kg.

Le tableau V rassemble les résultats des essais effectués avec les dérivés 1 et 3 de formule (I) et le composé de référence A et représentant les moyennes calculées à l'intérieur de chaque lot:

Tableau V

| Produits | Poids moyen des thrombi (mg) | | | | |
|---|---|---|---|---|---|
| | 12,5 mg/kg | 25 mg/kg | 50 mg/kg | 100 mg/kg | 200 mg/kg |
| Dérivé n° 1 | 27,45 | 12,21 | 6,01 | 4,76 | — |
| Dérivé n° 3 | 26,72 | 12,04 | 5,96 | 4,62 | — |
| Composé de référence A | 30,45 | 30,09 | 28,86 | 23,04 | 23,13 |
| Témoin gamme à 5% | 30,41 | | | | |

Ce test montre bien l'activité des composés de l'invention qui réduit considérablement le poids moyen des thrombi dès la dose de 25 mg/kg alors que le composé de référence A n'exerce aucune action anti-thrombotique, même à des doses élevées.

De plus, les tests ont montré que, contrairement aux composés des brevets n° 2.215.948 et 2.345.150, les dérivés de formule (I) sont totalement dépourvus de propriétés anti-inflammatoires et d'effets vasodilatateurs. Ils possèdent donc des propriétés beaucoup plus sélectives, ce qui leur confère un grand intérêt sur le plan thérapeutique où certaines activités complémentaires, quand elles ne sont pas recherchées, peuvent être préjudiciables pour le malade.

Les études toxicologique et pharmacologique qui viennent d'être rapportées ont mis en évidence la faible toxicité des composés de l'invention et leur bonne tolérance ainsi que leurs intéressantes propriétés inhibitrices de l'agrégation plaquettaire et anti-thrombotique qui les rendent très utiles en thérapeutique humaine et vétérinaire.

Le médicament de l'invention peut être présenté pour l'administration orale, sous forme de comprimés, comprimés dragéifiés capsules, gouttes, pour l'administration parentérale sous forme de soluté injectable et pour l'administration rectale, sous forme de suppositoires.

Chaque dose unitaire contient avantageusement de 0,010 g à 0,500 g, les doses administrables journellement pouvant varier de 0,010 g à 1,50 g de principe actif, en fonction de l'âge du patient et de la gravité de l'affection traitée.

On donnera, ci-dessous, à titre d'exemple non limitatif, quelques formulations pharmaceutiques du médicament de l'invention.

1. Comprimés dragéifiés

| | |
|---|---|
| Dérivé n° 1 | 0,050 g |
| Excipient: | |
| Lactose, cellulose microcristalline, stéarate de magnésium, colophane, gomme laque, gomme arabique, talc, gélatine officinale, cire blanche, érythrosine. | |

2. Comprimés

| | |
|---|---|
| Dérivé n° 3 | 0,075 g |
| Excipient: | |
| Cellulose microcristalline, saccharose, amidon de maïs, stéarate de magnésium. | |

3. Capsules

| | |
|---|---|
| Dérivé n° 4 | 0,050 g |
| Excipient: | |
| Amidon de blé, talc, lactose. | |

4. Soluté injectable

| | |
|---|---|
| Dérivé n° 7 | 0,100 g |
| Excipient: | |
| Solvant isotonique qsp 5 ml. | |

5. Suppositoires

| | |
|---|---|
| Dérivé n° 8 | 0,075 g |
| Excipient: | |
| Triglycérides semi-synthétiques. | |

Le médicament de l'invention est utilisé avec profit en médecine pour ses activités anti-agrégante plaquettaire et anti-thrombotique. Par ses propriétés inhibitrices au niveau de certaines fonctions plaquettaires qui peuvent intervenir dans le mécanisme de formation des thromboses artérielles et veineuses, il est indiqué dans le traitement et la prévention des troubles plaquettaires induits dans les circuits extracorporels, ou consécutifs aux complications de l'athérome.

**Revendications pour les Etats contractants BE CH DE GB IT LI LU NL SE**

1. Dérivé de tétrahydro-5,6,7,7a 4H-thiéno-(3,2-c) pyridinone-2 répondant à la formule générale suivante

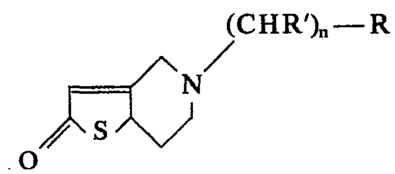

(I)

dans laquelle

R réprésente l'hydrogène ou un radical phényle éventuellement substitué par au moins un atome d'halogène ou un groupe alcoyle en $C_1$ à $C_4$, alcoxy en $C_1$ à $C_4$, nitro, carboxy, alcoxycarbonyle ou cyano;

R′ représente l'hydrogène ou un groupe alcoyle en $C_1$ à $C_4$; et
n est 0 ou un nombre entier de 1 à 4;

ainsi que leurs sels d'addition avec les acides minéraux ou organiques pharmaceutiquement acceptables.

2. Dérivés selon la revendication 1, caractérisés en ce que R est un radical phényle éventuellement substitué par un atome d'halogène, un radical alcoyle en $C_1$ à $C_4$, nitro ou cyano; R′ est un atome d'hydrogène ou un radical alcoyle en $C_1$ à $C_4$; et n est 1.

3. La o-chlorobenzyl-5-tétrahydro-5,6,7,7a-4H-thiéno (3,2-c)-pyridinone-2.

4. La benzyl-5-tétrahydro-5,6,7,7a-4H-thiéno-(3,2-c)-pyridinone-2.

5. La p-chlorobenzyl-5-tétrahydro-5,6,7,7a-4H-thiéno (3,2-c)-pyridinone-2.

6. La o-méthylbenzyl-5-tétrahydro-5,6,7,7a-4H-thiéno (3,2-c)-pyridinone-2.

7. La [1-(2-chlorophényl)-éthyl]-5-tétrahydro-5,6,7,7a-4H-thiéno (3,2-c)-pyridinone-2.

8. La [1-(2-chlorophényl)-propyl]-5-tétrahydro-5,6,7,7a-4H-thiéno (3,2-c)-pyridinone-2.

9. La o-cyanobenzyl-5-tétrahydro-5,6,7,7a-4H-thiéno (3,2-c)-pyridinone-2.

10. La o-nitrobenzyl-5-tétrahydro-5,6,7,7a-4H-thiéno (3,2-c)-pyridinone-2.

11. Procédé de préparation des dérivés de formule (I) selon l'une quelconque des revendications précédentes, caractérisé en ce que:

a) on oxyde un dérivé boronique de formule:

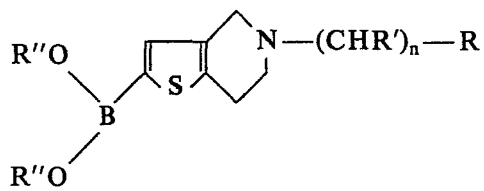

(V)

dans laquelle R, R′ et n ont les significations indiquées pour la formule (I) dans la revendication 1, et R″ est un atome d'hydrogène ou un groupe alcoyle inférieur; et

b) on hydrolyse le dérivé borique obtenu de formule suivante:

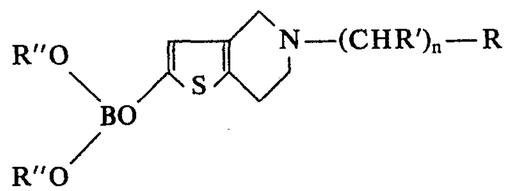

(VI)

dans laquelle: R, R′, R″ et n sont tels que définis ci-dessus, pour obtenir le dérivé de formule (I).

12. Procédé selon la revendication 11, caractérisé en ce que l'oxydation est effectuée par de l'eau oxygénée dans un solvant inerte et en empêchant une élévation de température du milieu réactionnel.

13. Procédé selon la revendication 11, caractérisé en ce que l'hydrolyse est réalisée par mise en contact du mélange réactionnel avec de l'eau.

14. Médicament ayant notamment des activités anti-agrégante plaquettaire et anti-thrombotique, caractérisé en ce qu'il contient à titre de princips actif un dérivé selon l'une quelconque des revendications 1 à 10.

15. Médicament selon la revendication 14, caractérisé en ce qu'il est présenté sous une forme appropriée à l'administration orale, parentérale ou rectale.

16. Médicament selon la revendication 14 ou 15, caractérisé en ce qu'il est présenté sous forme de doses unitaires contenant de 0,010 à 0,500 g de principe actif.

**Revendications pour l'Etat contractant AT**

1. Procédé de préparation de dérivés de la tétrahydro-5,6,7,7a 4H-thiéno (3,2-c) pyridinone-2 répondant à la formule générale suivante:

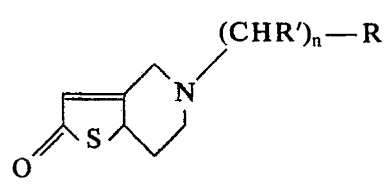

(I)

dans laquelle:

R représente l'hydrogène ou un radical phényle éventuellement substitué par ou moins un atome d'halogène ou un groupe alcoyle inférieur, alcoxy inférieur, nitro, carboxy, alcoxycarbonyle ou cyano;
R' représente l'hydrogène ou un groupe alcoyle inférieur; et
n est 0 ou un nombre entier de 1 à 4;

ainsi que leurs sels d'addition avec les acides minéraux ou organiques pharmaceutiquement acceptables, caractérisé en ce que:

a) on oxyde un dérivé boronique de formule:

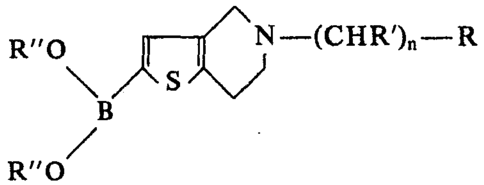

(V)

dans laquelle R, R' et n ont les significations indiquées ci-dessous, et R'' est un atome d'hydrogène ou un groupe alcoyle inférieur; et

b) on hydrolyse le dérivé borique obtenu de formule suivante:

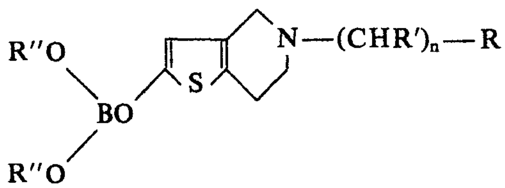

(VI)

dans laquelle: R, R', R'' et n sont tels que définis ci-dessus, pour obtenir le dérivé de formule (I).

2. Procédé selon la revendication 1, caractérisé en ce que l'oxydation est effectuée par de l'eau oxygénée dans un solvant inerte et en empêchant une élévation de température du milieu réactionnel.

3. Procédé selon la revendication 1, caractérisé en ce que l'hydrolyse est réalisée par mise en contact du mélange réactionnel avec de l'eau.

4. Procédé selon la revendication 1, caractérisé en ce que R est un radical phényle éventuellement substitué par un atome d'halogène, un radical alcoyle en $C_1$ à $C_4$, nitro ou cyano; R' est un atome d'hydrogène ou un radical alcoyle en $C_1$ à $C_4$; et n est 1.

**Patentansprüche für die Vertragsstaaten: BE, CH, DE, GB, IT, LI, LU, NL, SE**

1. 5,6,7,7a-Tetrahydro[4H]thieno(3,2-c)pyridin-2-onderivate der folgenden allgemeinen Formel:

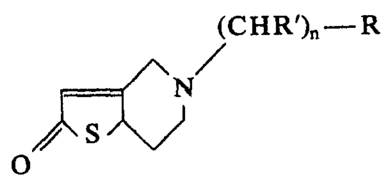

(I)

in der

R Wasserstoff oder ein gegebenenfalls mit wenigstens einem Halogenatom oder einer $C_1-C_4$-Alkyl-, $C_1-C_4$-Alkoxy-, Nitro-, Carboxy-, Alkoxycarbonyl oder Cyanogruppe substituierter Phenylrest,
R' Wasserstoff oder ein $C_1-C_4$-Alkylrest ist und
n für 0 oder eine ganze Zahl von 1 bis 4 steht,

sowie ihre Additionssalze mit pharmazeutisch annehmbaren Mineralsäuren oder organischen Säuren.

2. Derivate nach Anspruch 1, dadurch gekennzeichnet, daß R ein gegebenenfalls mit einem Halogenatom, einem $C_1-C_4$-Alkylrest, einer Nitro- oder Cyanogruppe substituierter Phenylrest, R' ein Wasserstoffatom oder ein $C_1-C_4$-Alkylrest und n 1 ist.

3. 5-o-Chlorobenzyl-5,6,7,7a-tetrahydro[4H]thieno(3,2-c)-pyridin-2-on.
4. 5-Benzyl-5,6,7,7a-tetrahydro[4H]thieno(3,2-c)pyridin-2-on.
5. 5-p-Chlorobenzyl-5,6,7,7a-tetrahydro[4H]thieno(3,2-c)pyridin-2-on.
6. 5-o-Methylbenzyl-5,6,7,7a-tetrahydro[4H]thieno(3,2-c)pyridin-2-on.
7. 5-[1-(2-Chlorophenyl)-ethyl]5,6,7,7a-tetrahydro[4H]thieno(3,2-c)pyridin-2-on.
8. 5-[1-(2-Chlorophenyl)-propyl]-5,6,7,7a-tetrahydro[4H]thieno(3,2-c)pyridin-2-on.
9. 5-o-Cyanobenzyl-5,6,7,7a-tetrahydro[4H]thieno(3,2-c)pyridin-2-on.
10. 5-o-Nitrobenzyl-5,6,7,7a-tetrahydro[4H]thieno(3,2-c)pyridin-2-on.

11. Verfahren zur Herstellung von Derivaten der Formel (I) nach irgendeinem der vorstehenden Ansprüche, dadurch gekennzeichnet, daß man

a) ein Boronsäurederivat der Formel

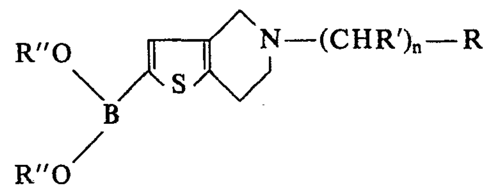

(V)

in der R, R' und n die für die Formel (I) in Anspruch 1 genannten Bedeutungen haben und R'' ein Wasserstoffatom oder ein niederer Alkylrest ist, oxidiert und

b) das erhaltene Borsäurederivat der folgenden Formel

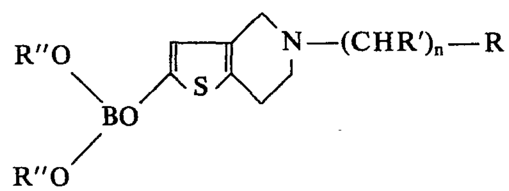

(VI)

in der R, R', R'' und n die vorstehend genannten Bedeutungen haben, hydrolysiert, um das Derivat der Formel (I) zu erhalten.

12. Verfahren nach Anspruch 11, dadurch gekennzeichnet, daß die Oxidation mit Wasserstoffperoxid in einem inerten Lösungsmittel und unter Verhinderung eines Temperaturanstiegs des Reaktionsmediums durchgeführt wird.

13. Verfahren nach Anspruch 11, dadurch gekennzeichnet, daß die Hydrolyse durch Inberührungbringen des Reaktionsgemisches mit Wasser durchgeführt wird.

14. Medikament mit insbesondere die Agglomeration der Blutplättchen hemmenden und antithrombotischen Eigenschaften, dadurch gekennzeichnet, daß es als Wirkstoff ein Derivat nach irgendeinem der Ansprüche 1 bis 10 enthält.

15. Medikament nach Anspruch 14, dadurch gekennzeichnet, daß es in einer für die orale, parenterale oder rektale Verabreichung geeigneten Form dargeboten wird.

16. Medikament nach Anspruch 14 oder 15, dadurch gekennzeichnet, daß es in Form von Einheitsdosen, die 0,001 bis 0,500 g Wirkstoff enthalten, dargeboten wird.

**Patentansprüche für den Vertragsstaat: AT**

1. Verfahren zur Herstellung von 5,6,7,7a-Tetrahydro[4H]thieno(3,2-c)pyridin-2-onderivaten der folgenden allgemeinen Formel:

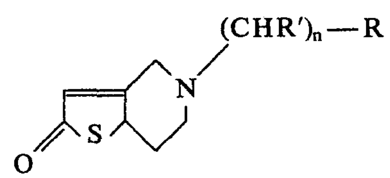

(I)

in der

R Wasserstoff oder ein gegebenenfalls mit wenigstens einem Halogenatom oder einer niederen Alkylgruppe, einer niederen Alkoxygruppe, Nitro-, Carboxy-, Alkoxycarbonyl- oder Cyanogruppe substituierter Phenylrest,

R' Wasserstoff oder ein niederer Alkylrest und
n für 0 oder eine ganze Zahl von 1 bis 4 steht,

sowie ihre Additionssalze mit pharmazeutisch annehmbaren Mineralsäuren oder organischen Säuren, dadurch gekennzeichnet, daß man

a) ein Boronsäurederivat der Formel

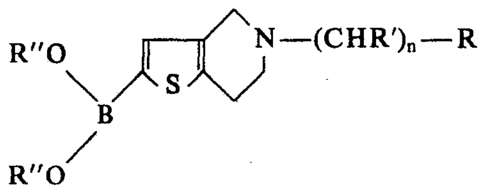

(V)

in der R, R' und n die vorstehend genannten Bedeutungen haben und R'' ein Wasserstoffatom oder ein niederer Alkylrest ist, oxidiert und

b) das erhaltene Borsäurederivat der folgenden Formel:

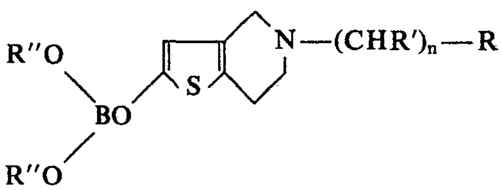

(VI)

in der R, R', R'' und n die vorstehend genannten Bedeutungen haben, hydrolysiert,

um das Derivat der Formel (I) zu erhalten.

2. Verfahren nach Anspruch 1, dadurch gekennzeichnet, daß die Oxydation mit Wasserstoffperoxid in einem inerten Lösungsmittel und unter Verhinderung eines Temperaturanstiegs des Reaktionsmediums durchgeführt wird.

3. Verfahren nach Anspruch 1, dadurch gekennzeichnet, daß die Hydrolyse durch Inberührungbringen des Reaktionsgemisches mit Wasser durchgeführt wird.

4. Verfahren nach Anspruch 1, dadurch gekennzeichnet, daß R ein gegebenenfalls mit einem Halogenatom, einem $C_1-C_4$-Alkylrest, einer Nitro- oder Cyanogruppe substituierter Phenylrest, R' ein Wasserstoffatom oder ein $C_1-C_4$-Alkylrest und n 1 ist.

**Claims for the contracting states: BE, CH, DE, GB, IT, LI, LU, NL, SE**

1. Derivatives of 5,6,7,7a-tetrahydro-4H-thieno[3,2,c]pyridin-2-one of the general formula:

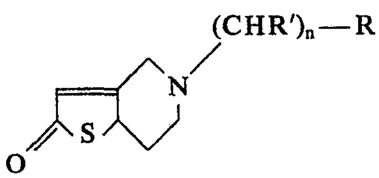

(I)

in which

R is a hydrogen or a phenyl radical which is optionally substituted by at least one halogen atom or $C_1-C_4$ alkyl group, $C_1-C_4$ alcoxy, nitro, carboxy, alkoxycarbonyl or cyano;
R' is a hydrogen or a $C_1-C_4$ alkyl group; and
n is 0 or an integer from 1 to 4;

and the addition salts thereof with pharmaceutically acceptable mineral or organic acids.

2. Derivatives according to claim 1, wherein R is a phenyl radical optionally substituted by a halogen atom, a $C_1-C_4$ alkyl, nitro or cyano; R' is a hydrogen atom or a $C_1-C_4$ alkyl radical; and n is 1.

3. 5-(o-Chlorbenzyl)-5,6,7,7a-tetrahydro-4H-thieno[3,2-c]pyridin-2-one.

4. 5-Benzyl-5,6,7,7a-tetrahydro-4H-thieno[3,2-c]pyridin-2-one.

5. 5-(p-Chlorobenzyl)-5,6,7,7a-tetrahydro-4H-thieno[3,2-c]pyridin-2-one.

6. 5-(o-Methylbenzyl)-5,6,7,7a-tetrahydro-4H-thieno[3,2-c]pyridin-2-one.

7. 5-[1-(2-Chlorophenyl)-ethyl]-5,6,7,7a-tetrahydro-4H-thieno[3,2-c]pyridin-2-one.

8. 5-[1-(2-Chlorophenyl)-propyl]-5,6,7,7a-tetrahydro-4H-thieno[3,2-c]pyridin-2-one.
9. 5-(o-Cyanobenzyl)-5,6,7,7a-tetrahydro-4H-thieno[3,2-c]pyridin-2-one.
10. 5-(o-Nitrobenzyl)-5,6,7,7a-tetrahydro-4H-thieno[3,2-c]pyridin-2-one.
11. Process for the preparation of compounds according to claim 1, wherein

a) a boronic acid derivative of the general formula:

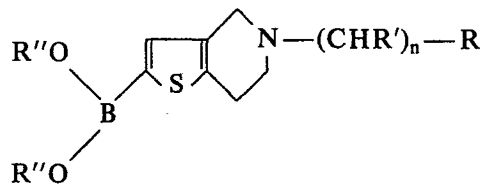

(V)

in which R, R' and n have the meanings given for formula (I) in claim 1 and R'' is a hydrogen atom or a lower alkyl radical, is oxidised, and

b) the boric acid derivative obtained of the general formula:

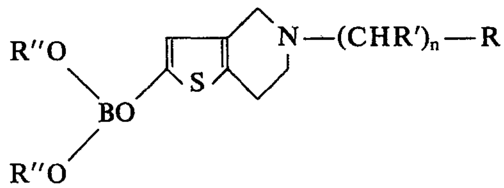

(VI)

in which R, R', R'' and n have the same meanings as above, is hydrolysed to give a compound of general formula (I).

12. Process according to claim 11, wherein the oxidation is carried out with hydrogen peroxide in an inert solvent under conditions which prevent a rise in temperature of the reaction mixture.
13. Process according to claim 11, wherein the hydrolysis is carried out by contacting the reaction mixture with water.
14. A medicament particularly having anti-platelet aggregation and anti-thrombosis activities, comprising one derivative according to any one of the claims 1 to 10 as active principle.
15. A medicament according to claim 14, whenever in a form appropriate for oral, parenteral or rectal administration.
16. A medicament according to claim 14 or 15, whenever present in the form of a dosage unit containing from 0.010 to 0.500 g of active principle.

**Claims for the contracting state: AT**

1. Process for the preparation of derivatives of 5,6,7,7a-tetrahydro-4H-thieno[3,2,c]pyridin-2-one of the general formula:

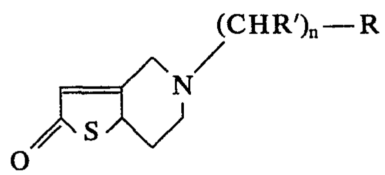

(I)

in which

R is a hydrogen or a phenyl radical, which is optionally substituted by at least one halogen atom or $C_1-C_4$ alkyl radical, $C_1-C_4$ alkoxy, nitro, carboxyl, alkoxycarbonyl or cyano;
R' is a hydrogen or a $C_1-C_4$ alkyl group; and
n is 0 or an integer of 1 to 4;

and the addition salts thereof with pharmaceutically acceptable mineral or organic acids, wherein

a) a boronic acid derivative of the general formula:

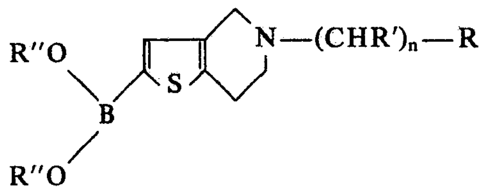

(V)

in which R, R' and n have the same meanings as above and R'' is a hyrogen atom or a lower alkyl radical, is oxidised, and

b) the boric acid derivative obtained of the general formula:

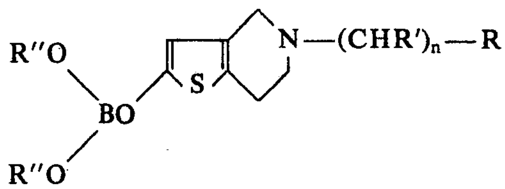

(VI)

in which R, R', R'' and n have the same meanings as above, is hydrolysed to give a compound of general formula (I).

2. Process according to claim 1, wherein the oxidation is carried out with hydrogen peroxide in an inert solvent under conditions which prevent a rise in temperature of the reaction mixture.

3. Process according to claim 1, wherein the hydrolysis is carried out by contacting the reaction mixture with water.

4. Process according to claim 1, wherein R is a phenyl radical, which is optionally sustituted by a halogen atom, a $C_1-C_4$ alkyl radical, nitro or cyano; R' is a hydrogen atom or a $C_1-C_4$ alkyl radical; and n is 1.